(19) Europäisches Patentamt
European Patent Office
Office européen des brevets

(11) **EP 4 429 543 B1**

(12) **EUROPEAN PATENT SPECIFICATION**

(45) Date of publication and mention
of the grant of the patent:
**24.12.2025 Bulletin 2025/52**

(21) Application number: **22821317.9**

(22) Date of filing: **11.11.2022**

(51) International Patent Classification (IPC):
*A61B 5/145* (2006.01)     *A61B 10/00* (2006.01)

(52) Cooperative Patent Classification (CPC):
**A61B 5/4318; A61B 5/14507; A61B 10/0012**

(86) International application number:
**PCT/EP2022/081633**

(87) International publication number:
**WO 2023/084030 (19.05.2023 Gazette 2023/20)**

(54) **METHOD FOR DETERMINATION OF THE WINDOW OF IMPLANTATION**

VERFAHREN ZUR BESTIMMUNG EINES IMPLANTATIONSFENSTERS

PROCÉDÉ DE DÉTERMINATION DE LA FENÊTRE D'IMPLANTATION

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **12.11.2021 EP 21383030**

(43) Date of publication of application:
**18.09.2024 Bulletin 2024/38**

(73) Proprietors:
  • **Manina Medtech, S.L.**
    **08010 Barcelona (ES)**
  • **Fundació Hospital Universitari Vall d'Hebron - Institut de Recerca**
    **08035 Barcelona (ES)**

(72) Inventors:
  • **RODRIGUEZ DE LA VEGA OTAZO, Monica**
    **08010 Barcelona (ES)**
  • **GALLART AGUT, Roger**
    **08004 Barcelona (ES)**
  • **CARBONELL SOCIAS, Melchor**
    **08035 Barcelona (ES)**
  • **HERRERO GARCIA, Julio**
    **08328 Alella- Barcelona (ES)**
  • **ARTILES MARTINEZ, Luis Manuel**
    **08010 Barcelona (ES)**
  • **TEIXIDO TROYANO, Anna**
    **08035 Barcelona (ES)**

(74) Representative: **ABG Intellectual Property Law, S.L.**
**Avenida de Burgos, 16D**
**Edificio Euromor**
**28036 Madrid (ES)**

(56) References cited:
**WO-A1-2021/028562**

• **TRANQUILLI A L ET AL: "The origin of pre-eclampsia: From decidual "hyperoxia" to late hypoxia", MEDICAL HYPOTHESES, EDEN PRESS, PENRITH, US, vol. 75, no. 1, 1 July 2010 (2010-07-01), pages 38 - 46, XP027062813, ISSN: 0306-9877, [retrieved on 20100218], DOI: 10.1016/ J.MEHY.2010.01.024**
• **KEA ET AL: "Effect of reduced oxygen concentrations on the outcome of in vitro fertilization", FERTILITY AND STERILITY, ELSEVIER, AMSTERDAM, NL, vol. 87, no. 1, 29 December 2006 (2006-12-29), pages 213 - 216, XP005818556, ISSN: 0015-0282, DOI: 10.1016/ J.FERTNSTERT.2006.05.066**
• **BAVISTER ET AL: "Oxygen concentration and preimplantation development", REPRODUCTIVE BIOMEDICINE ONLINE, ELSEVIER, AMSTERDAM, NL, vol. 9, no. 5, 1 January 2004 (2004-01-01), pages 484 - 486, XP027050136, ISSN: 1472-6483, [retrieved on 20040101]**

**(Cont. next page)**

- HARVEY ET AL: "The role of oxygen in ruminant preimplantation embryo development and metabolism", ANIMAL REPRODUCTION SCIENCE, ELSEVIER SCIENCE PUBLISHERS, AMSTERDAM, NL, vol. 98, no. 1-2, 9 February 2007 (2007-02-09), pages 113 - 128, XP005880891, ISSN: 0378-4320, DOI: 10.1016/J.ANIREPROSCI.2006.10.008

**Description**

**FIELD OF THE INVENTION**

The present invention relates to the field of assisted reproduction technologies and, more in particular, to methods based on analysis of dissolved gases for determining endometrial receptivity in a female subject and methods for selecting a female subject as a candidate to receive an embryo.

**BACKGROUND OF THE INVENTISON**

Infertility is a global health problem affecting around 190 million people worldwide. Among Assisted Reproduction Techniques (ART), In Vitro Fertilization (IVF) is one of the preferred treatments, where the embryo is cultivated in the lab and then transferred into the future mother's uterus. On average, a woman needs three complete IVF cycles to get pregnant. More than 900 000 cycles per year are done in Europe but only 35% result in pregnancy. One of the leading causes of failure is embryo transfer, with a rate of live birth per embryo transfer below 30%.

The global IVF market is expected to reach USD 37.7 billion by 2027, expanding at a compound annual growth rate (CAGR) of 9.5%. Europe dominated the procedure and instrument market for IVF in 2019. Japan, USA and Spain were the most active countries globally and the demand in Asia-Pacific is expected to boom. The potential clients are private and public IVF clinics, as the product aims to be used by healthcare professionals. There are 1200 clinics in Europe (250 in Spain) and 480 clinics in the USA.

Embryo quality, uterine receptivity, and embryo-uterine synchrony are critical for successful implantation. Nowadays, it is possible to assess embryo maturation and quality, but the estimation of endometrial development and synchrony is highly imprecise because it relies on counting the days after ovulation or the days of progesterone treatment. The period of time during which the endometrium is receptive is called Window of Implantation (WOI), and it usually occurs during the luteal phase, between days 19 and 21 of the menstrual cycle. Its duration and position in the cycle depend on various factors and can vary from woman to woman and from month to month in the same woman, making it challenging to determine empirically. Accuracy in endometrial receptivity detection is essential for every woman but critical for women having displaced or shortened WOI, which accounts for 30% of all IVF patients. This percentage is even higher in women with repeated implantation failure. Ultrasound/Doppler techniques to check the endometrium thickness and vascularity have failed to measure accurately endometrial receptivity and synchrony, and no useful markers for endometrial receptivity have been found using histology approaches. The arrival of "omics" technologies made allowed the large scale analysis of the expression of genes, proteins, microRNAs, lipid metabolism, and secretory pattern of the receptive endometrium. Commercial tests focused on detecting the gene expression profile that characterizes the receptive endometrium are available, for example ERA, ERPeak, Le Win-Test, ER Map. These methods are invasive, require an endometrial biopsy and several days to obtain the results, making it impossible to perform the test and the transfer in the same cycle.

Further methods for determination of endometrial receptivity are known from TRANQUILLI A L ET AL: "The origin of pre-eclampsia: From decidual "hyperoxia" to late hypoxia", MEDICAL HYPOTHESES, EDEN PRESS, PENRITH, US, vol. 75, no. 1, July 2010, pages 38-46, ISSN: 0306-9877, DOI: 10.1016/J.MEHY.2010.01.024, KEA ET AL: "Effect of reduced oxygen concentrations on the outcome of in vitro fertilization", FERTILITY AND STERILITY, ELSEVIER, AMSTERDAM, NL, vol. 87, no. 1, December 2006, pages 213-216, ISSN: 0015-0282, DOI: 10.1016/J.FERTNSTERT.2006.05.066, BAVISTER ET AL: "Oxygen concentration and preimplantation development", REPRODUCTIVE BIOMEDICINE ONLINE, ELSEVIER, AMSTERDAM, NL, vol. 9, no. 5, January 2004, pages 484-486, ISSN: 1472-6483, HARVEY ET AL: "The role of oxygen in ruminant preimplantation embryo development and metabolism", ANIMAL REPRODUCTION SCIENCE, ELSEVIER SCIENCE PUBLISHERS, AMSTERDAM, NL, vol. 98, no. 1-2, February 2007, pages 113-128, ISSN: 0378-4320, DOI: 10.1016/ J.ANIREPROSCI.2006.10.008, and WO-2021/028562-A1.

There is a need for improving timely detection of endometrial receptivity, coordinate embryo transfer with uterine development, and match embryo-uterine synchrony to increase success rates of assisted reproduction techniques. There is also a need for facilitating the identification of women who are eligible to receive an embryo to increase the live birth rates per embryo transfer and the success of the assisted reproduction procedure, making it shorter, less stressful, and more affordable.

**SUMMARY OF THE INVENTION**

The authors of the present invention have found a novel method for the determination of the state of uterine receptivity, in particular, the window of implantation (WOI) for embryo transfer in a female subject. The WOI manifests by the occurrence of a receptive endometrium in said female subject and, according to the present invention, it can be determined by measuring the dissolved gas concentration in the endometrial fluid of said female subject. It is estimated that more than 30% of women have a displaced or shortened window of implantation, which usually lasts less than 48 hours, and this percentage is even higher in women with repeated implantation failures. There are no validated biomarkers or anatomical indicators for the detection of the window of implantation. Methods for gene expression analysis are widely used for determination of endometrial receptivity but they require the use of sophisticated equipment and the collection of tissue biopsies. The authors have identified that selecting $O_2$ and/or $CO_2$ as the dissolved gas to be measured in endometrial fluid of a female subject allows for a technically simple but highly reliable determination of endometrial receptivity despite the difficulties associated to the variations from cycle to cycle and with respect to the physiological differences among women.

Therefore, in a first aspect the invention relates to a method for determination of endometrial receptivity in a female subject, the method comprising the steps of:

i) measuring the endometrial fluid dissolved gas concentration of said female subject, thereby obtaining a dissolved gas concentration value, wherein the dissolved gas is selected from the group consisting of $O_2$ and $CO_2$,

ii) comparing said dissolved gas concentration value with a reference value, wherein an increase of the concentration value of dissolved $O_2$ and/or a decrease in the concentration value of dissolved $CO_2$ with respect to the reference value is indicative that the endometrium of said female subject is receptive.

The authors have also found that measuring the dissolved gas concentration in the endometrial fluid of a female subject allows for the selection of a female subject undergoing an *assisted reproduction technique (ART)* as a candidate to receive an embryo.

Therefore, in a second aspect, the present invention relates to a method for selecting a female subject undergoing an assisted reproduction procedure as a candidate to receive an embryo, the method comprising the steps of:

i) determining whether the endometrium of said female subject is receptive following the method steps according to the invention, and if said endometrium is receptive,

ii) selecting said female subject as a candidate to receive an embryo in an embryo transfer procedure to be performed on the same day as step i).

## BRIEF DESCRIPTION OF THE FIGURES

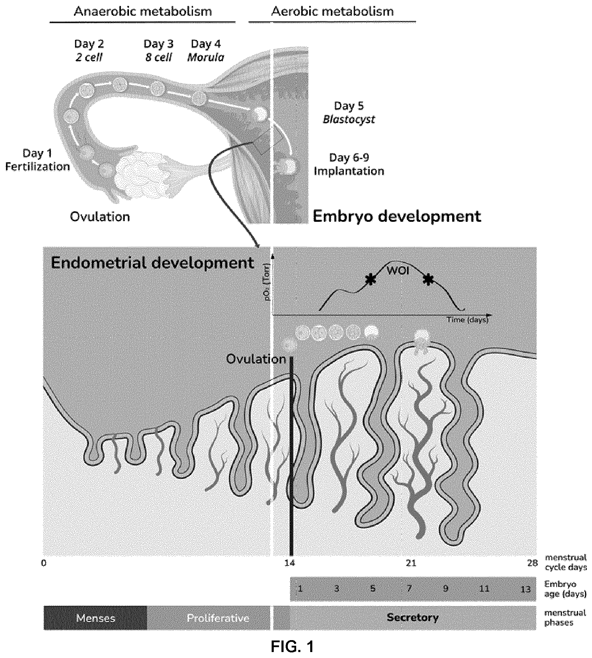

**Figure 1.** Schematic representation of the principle of embryo/endometrium synchrony according to the invention showing the chronological correlation of the development stages of the embryo with respect to the window of implantation and in relation to the intrauterine oxygen concentration.

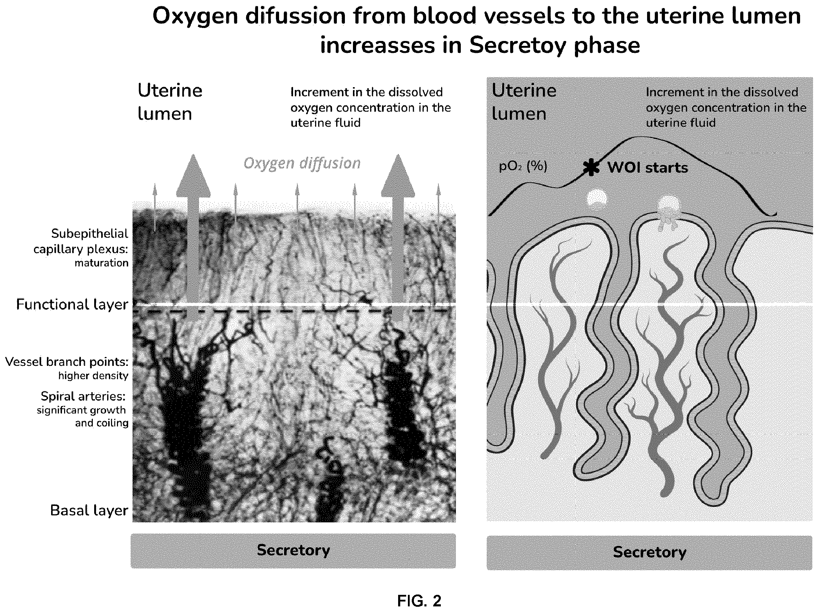

**Figure 2.** Schematic representation of the principle of the invention showing the oxygen diffusion from the uterus into the uterine lumen and the increment of the dissolved intrauterine oxygen concentration in the uterine fluid at the start of the window of implantation. Image from Bartelmez GW. The form and the functions of the uterine blood vessels in the rhesus monkey. Contrib Embryol 1957;36:154-83, courtesy of Carnegie Institution for Science, Copyright Carnegie Institution for Science.

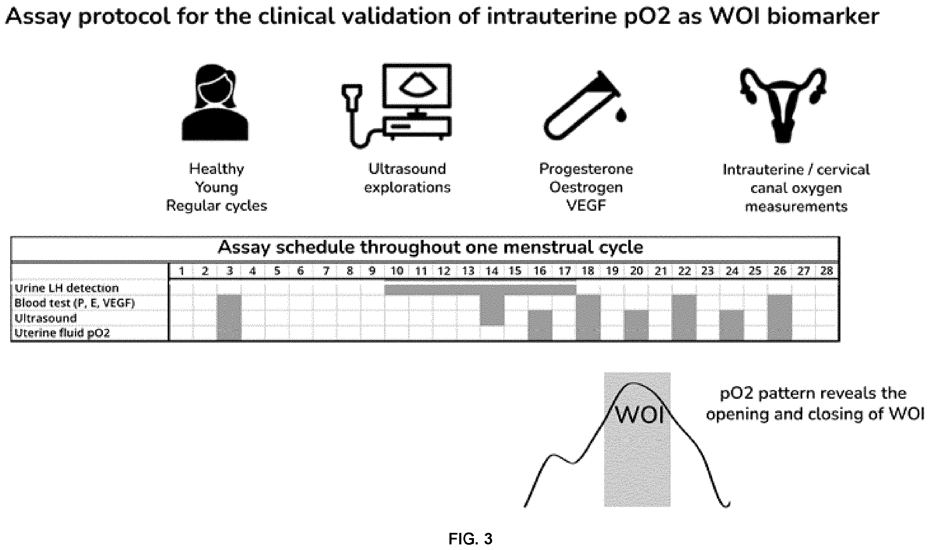

**Figure 3.** Schematic representation of the principle of the invention showing the correlation of the variation of pO2 and the occurrence of the window of implantation including additional testing scheduled throughout the menstrual cycle.

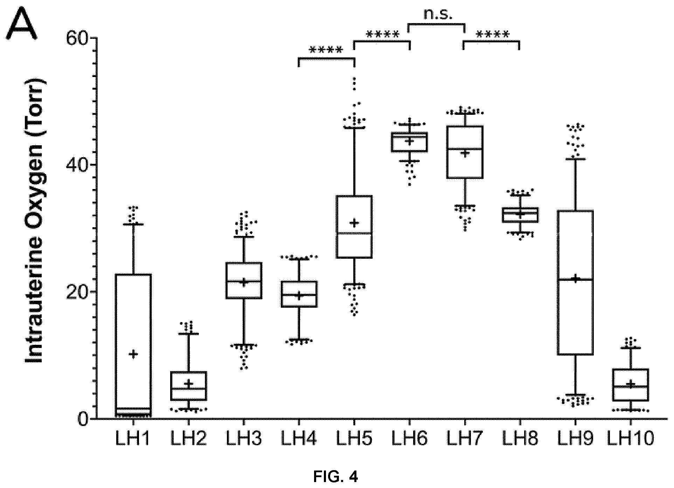

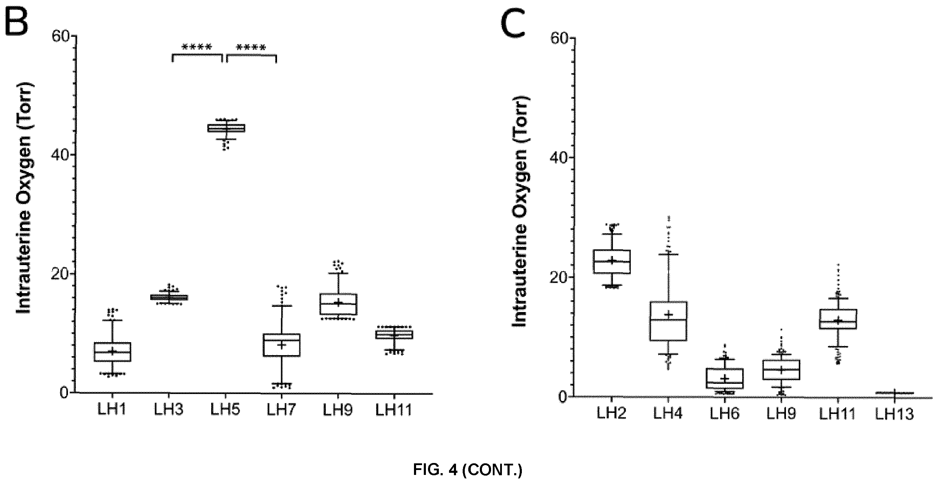

**Figure 4.** Intrauterine dissolved oxygen patterns observed during the luteal phase of the women in the study. Dissolved oxygen concentration was measured with a sterilized IMP-7 optical sensor mounted on a transfer catheter. Measurements were carried out every second for five minutes, and the first 120 seconds were filtered out due to lag time. Box and whiskers graphs were used. The whiskers represent 5-95% of the data; the median is represented by a line and the mean with a + symbol. Data outside whiskers are represented as dots. Oxygen concentration is expressed in Torr, and 1 Torr = 133,22 Pa. **A:** The data from three different patients were combined. The oxygen level significantly increases from LH4 to LH5 and from LH5 to LH6 ($p<0.0001$, Tukey's multiple comparisons test). There are no differences between LH6 and LH7, and $pO_2$ significantly decreases in LH8 ($p<0.0001$). See Tables 1-3. The Window of Implantation (WOI) occurs in LH6-LH7 of natural cycles (or P5-P6 of artificial cycles). The $pO_2$ pattern in A represents the standard of WOI occurrence and describes the WOI's opening (LH5) and closing (LH8). **B:** Data represent the dissolved intrauterine $pO_2$ pattern of a volunteer having an advanced and shortened WOI: the $pO_2$ maximum in LH5 and a minimum in LH7 (See Tables 1-3). C: Data represent a healthy volunteer's dissolved intrauterine $pO_2$ profile with no oxygen level increase during the luteal phase.

## DETAILED DESCRIPTION OF THE INVENTION

Under ideal conditions, the state of endometrial receptivity, called the Window of Implantation (WOI), usually occurs during the luteal phase, between days 19 and 21 of the menstrual cycle (Navot D. et al., Fertil Steril 1991, 55:114-118; Harper MJK. Baillieres Clin Obstet Gynaecol 1992, 6: 351-371). In artificial cycles (hormonereplacement therapy cycles), the window of implantation occurs after five days of progesterone treatment ($P_4$+5) (Enciso, M. et al., Sci Rep 11, 13420 (2021)). Its duration and position in the cycle depend on several factors and can vary from month to month in the same woman, making it difficult to estimate empirically. In fact, more than 30% of women have displaced or shortened WOI, this percentage being even higher in women with repeated implantation failures. Despite the efforts of many research groups, methods of detecting WOI through anatomical patterns or molecular biomarkers have not been established. The advent of "omics" technologies in recent decades has enabled the development of new diagnostic tests focused on detecting the gene expression profile that characterizes WOI. However, these methods require a biopsy of endometrial tissue and at least one week to obtain the results, making it impossible to perform the test during the active ART cycle. In addition, "omics" technologies rely on the use of sophisticated technology and/or the services of "omics" analysis providers. In other word, in most cases "omics" technology cannot be performed by the clinician himself. Moreover, the results are a prediction of the position of WOI in a future cycle, and to increase the accuracy of this prognosis, artificial preparation of the endometrium using exogenous hormones is recommended. It is important to highlight that artificially prepared cycles have more obstetric and perinatal complications than natural cycles, constituting not only a risk for the health of the mother and fetus, but also a higher expense for the healthcare system, so it is a priority for ART services to facilitate the use of methods as close as possible to the natural cycle, if the patient's physiology allows it. The present invention overcomes the above problems.

Oxygen homeostasis is critical for eukaryotes' survival. Increases in cellular oxygen levels result in the generation of toxic oxygen species, and oxygen absence provokes mitochondrial respiration inhibition. In particular, the embryo is exposed to the oxygen dissolved in the uterine or endometrial fluid regulated by the uterine system through endometrial thickening and angiogenesis and has to be tightly coordinated with embryo metabolic requirements. In fact, embryo metabolism changes from anaerobic to aerobic in only five days.

The authors of the present invention have developed a method by which the identification of the window of implantation, manifested by the occurrence of a receptive endometrium, is associated with the concentration of dissolved gas in endometrial fluid. The authors provide evidence that the change in trend of the dissolved gas concentration in endometrial fluid throughout the menstrual cycle allows for the elucidation of the suitable timing (embryo/endometrium synchrony) for embryo implantation in a given female subject. The authors have also found that the uterine environment is hypoxic during the anaerobic phases of the embryo and when endometrium becomes receptive, the intrauterine oxygen pressure increases, coincident with blastocyst development and implantation, during which embryo oxygen consumption increases dramatically.

Accordingly, the present invention provides a method for determination of endometrial receptivity in a female subject and, thus, a method for identifying the window of implantation as well as for assessing embryo implantation outcome and pregnancy. The method of the present invention provides reliable determination of endometrial receptivity with the advantage that obtaining an endometrial biopsy is no longer necessary. Notably, the present method allows the clinicians both to identify the window of implantation and to perform embryo transfer within hours, i.e. in the same day. The method of the invention does not require costly or bulky equipment, but handheld standard, medical gas measuring devices, so that it can be applied by a wide range of clinics, even those with lower budgets. Also, the simple and fast identification of the window of implantation reduces the need of administering unnecessary hormonal treatments so as to make the prediction of WOI in upcoming cycles more accurate, which is associated to more frequent pregnancy complications with respect to natural cycles. Accordingly, this method finds application in assisted reproduction centers and *in vitro* fertilization units for improving rates of embryo implantation and pregnancy, accelerating the clinical decision process, as well as reducing costs and complications derived from the administration of hormonal treatments in women that can benefit from natural cycles.

### 1. *Method for determination of endometrial receptivity in a female subject*

The authors of the present invention have found that determining the concentration of dissolved gas in endometrial fluid in a female subject makes the reliable identification of the window of implantation in said female subject possible. Thus, in a first aspect of the invention, the invention relates to a method for determination of endometrial receptivity in a female subject (first method of the invention), the method comprising the steps of

i) measuring the endometrial fluid dissolved gas concentration of said female subject, thereby obtaining a dissolved gas concentration value, wherein the dissolved gas is selected from the group consisting of $O_2$ and $CO_2$,
ii) comparing said dissolved gas concentration value with a reference value, wherein an increase of the concentration

value of dissolved $O_2$ and/or a decrease in the concentration value of dissolved $CO_2$ with respect to the reference value is indicative that the endometrium of said female subject is receptive

In a first step, the endometrial fluid dissolved gas concentration of said female subject is measured, thereby obtaining a dissolved gas concentration value.

The expression "determining the endometrial receptivity" as used herein relates to the assessment or prediction of a physiological or clinical status of the endometrium, in particular, with regards to the pregnancy outcome (e.g. likelihood of pregnancy or implantation-capable embryo survival).

The term "endometrial receptivity", as used herein, relates to the state in which a female subject's endometrium is prepared for embryo implantation. The term "endometrial receptivity" may refer to the status during a period of time in which the endometrium acquires a functional status that allows blastocyst implantation and leads to pregnancy. Implantation consists of three stages: (1) apposition, when the blastocyst contacts the implantation site; (2) adhesion, when blastocyst attach to the receptive endometrial epithelium; and (3) invasion, when the invasive trophoblast cells of the blastocyst cross the endometrial epithelial and invade the endometrial stroma. The term "window of implantation" may refer to the period of time during which the uterus of said female subject is receptive for embryo implantation. The term "receptive endometrium" may also refer to the status of the endometrium which is characteristic of the period of time known as "window of implantation" in a female subject. The term "endometrial receptivity" may also refer to the stage of an endometrium at which the window of implantation takes place so that an embryo transfer leads to pregnancy with high probability.

The term "subject" or "individual" or "animal" or "patient" or "mammal" is understood as any subject, particularly a mammalian subject, for whom determination or diagnosis or prognosis is desired. Mammalian subjects include humans, domestic animals, farm animals, and zoo, sports, or pet animals such as dogs, cats, guinea pigs, rabbits, rats, mice, horses, cattle, cows, and so on. In a preferred embodiment of the invention, the subject is a mammal. In a more preferred embodiment of the invention, the subject is a human.

As used herein the term "female subject" refers to a mammalian female whose endometrial receptivity is to be determined. Typically said mammal is a human (i.e. a woman), but may concern other mammals such as primates, dogs, cats, pigs, sheep, cows.

The endometrium is the inner epithelial layer, along with its mucous membrane, of the mammalian uterus, forming a cavity. As used herein, the term "endometrial fluid" (EF) or "endometrial cavity fluid" (ECF) or "uterine fluid" or "uterine cavity fluid" is a fluid accumulation within the endometrial cavity.

As used herein the term "dissolved gas" may refer to the gas tension in a fluid. The term "gas tension" may refer to the partial pressure of gases in said fluid. In a mixture of gases, each constituent gas has a "partial pressure" which is the notional pressure of that constituent gas if it alone occupied the entire volume of the original mixture at the same temperature. In the context of the invention, the amount of a given gas dissolved in a given liquid, in particular endometrial fluid, is directly proportional to the partial pressure of the gas in contact with the liquid. As used herein, the partial pressure of a gas in a solvent is also proportional to the concentration of the gas dissolved in the given liquid, in particular endometrial fluid. In the context of the present invention the term "dissolved gas concentration", "gas tension", "partial pressure" or "partial tension" are used interchangeably. Methods for measuring the "dissolved gas concentration" are known in the art.

According to the present invention, the method comprises measuring the oxygen ($O_2$) tension ($PxO_2$) and/or carbon dioxide ($CO_2$) tension ($PxCO_2$). Conventionally, the subscript "x" in each symbol represents the tissue or organ comprising the fluid in which the dissolved gas is measured: for example, "a" meaning arterial (blood), "v" being venous (blood), "c" being capillary (blood). In some embodiments, $PuO_2$ "u" meaning uterine fluid or $PeO_2$ "e" meaning endometrial fluid are used interchangeably and may refer to the oxygen tension in the fluid comprised in the endometrial cavity. In some embodiments, $PuCO_2$ "u" meaning uterine fluid or $PeCO_2$ "e" meaning endometrial fluid are used interchangeably and may refer to the carbon dioxide tension in the fluid comprised in the endometrial cavity.

According to the present invention, partial pressure of oxygen in endometrial fluid may be expressed in mmHg units. In some embodiments, the partial pressure of oxygen is interchangeably expressed as a concentration value in percentage (%) of dissolved oxygen. In some embodiments, partial pressure of oxygen in endometrial fluid may be expressed in kPa units or Torr. Methods for unit conversion are known in the art. For example, it is well-known that one Torr is 1001325/760 pascals, therefore approximately 133.22 Pa.

According to the present invention, partial pressure of carbon dioxide in endometrial fluid may be expressed in mmHg units. In some embodiments, the partial pressure of carbon dioxide is interchangeably expressed as a concentration value in percentage (%) of dissolved carbon dioxide. In some embodiments, partial pressure of carbon dioxide in endometrial fluid may be expressed in kPa units.

According to the present invention, measuring the endometrial fluid dissolved gas concentration refers to the absolute or relative determination of the concentration of gas dissolved in endometrial fluid by means of any conventional measuring device suitable to detect and quantify the presence of a gas dissolved in a fluid. Non limiting examples of techniques suitable for measuring the concentration of dissolved gas are conventional devices used in medicine in the analysis of arterial/venous blood and analysis of respiratory air, polarographic microelectrodes, electrochemical electrodes, optical sensors, fiber-optic sensors, fluorophore quenching-based sensors. Further non limiting examples of techniques suitable for measuring the concentration of dissolved gas include dynamic contrast-enhanced magnetic resonance imaging, blood oxygen leveldependent imaging and electron paramagnetic resonance imaging. Such sensors are well known in the art and can be readily applied by those skilled in the art in order to carry out the measuring functions associated with the present invention.

In some embodiments, the dissolved gas concentration is measured optically, preferably by phase fluorometry. The term "optical method", as used herein, refers to any method wherein light is used for the detection, characterization and/or quantification of a gas. Optical methods can be based on measuring absorbance, fluorescence intensity or fluorescence lifetime. Illustrative non-limitative examples of optical methods that can be used for measuring $O_2$ and $CO_2$ dissolved concentration are usually based on the quenching of fluorescence in the presence of oxygen. They include intensity quenching detection or lifetime quenching detection, both in the time or the frequency domains. Methods for measuring and analyzing time-resolved fluorescence traces include phasemodulation fluorometry or pulse fluorometry (direct emission decay measurements, single-photon timing, streak camera measurements, fluorescence upconversion, and optical Kerr gating). Optical methods and fluorophores for measuring the dissolved gas concentration are known in the art.

The term "phase fluorometry", as used herein, refers to a method in which the sample is excited by light sinusoidally modulated at high frequency. The fluorescence response of the dye is also sinusoidally modulated at the same frequency but is timedelayed or phase-shifted relative to the excitation signal, which in turn depends on the $pO_2$ in contact with the dye in the surface of the sensor probe.

As used herein, measuring the endometrial fluid dissolved gas concentration may refer to the determination of oxygen tension in endometrial fluid. As used herein, measuring the endometrial fluid dissolved gas concentration may refer to the determination of carbon dioxide tension in endometrial fluid. As used herein, measuring the endometrial fluid dissolved gas concentration may refer to the determination of oxygen tension and carbon dioxide tension in endometrial fluid.

In a second step of the first method of the invention for determining endometrial receptivity in a female subject, the endometrial fluid dissolved gas concentration value is compared to a reference value.

The term "reference value" or "reference level", as used herein in the context of the methods of the invention, relates to a value used as a reference for the values obtained from measurement of concentration of dissolved gas to be classified within a certain physiological or clinical status or to be classified as having high or low probability of entering a physiological or clinical status, or to be classified as having good or bad prognosis for entering a physiological or clinical status. The reference value or reference level according to any of the methods of the invention can be an absolute value; a relative value; a value that has an upper and/or lower limit; a range of values; an average value; a median value, a mean value, or a value as compared to a particular control or baseline value. A reference value can be based on an individual sample value, for example, a value obtained from the female subject being tested, however, at an earlier point in time than the measurement under consideration, which is to be compared with said reference value. The reference value can be based on a large number of samples, for example, from a population of subjects of the chronological age matched group, or based on a pool of samples including or excluding the sample to be tested. Various considerations are taken into account when determining the reference value of the marker. Among such considerations are the time point of the menstrual cycle, age, weight, sex, general physical condition of the patient and the like. For example, equal amounts of a group of at least 2, at least 10, at least 100 to preferably more than 1000 subjects, preferably classified according to the foregoing considerations, for example according to various age categories, are taken as the reference group.

The term "reference value" as used herein, may refer to the concentration value of dissolved gas in endometrial fluid under consideration in a given subject at a given time point. In a preferred embodiment, the reference value corresponds to the concentration value of $O_2$ in endometrial fluid at a given time point. In other embodiments, the reference value corresponds to the concentration value of $CO_2$ in endometrial fluid at a given time point. In other embodiments, the reference value corresponds to the individual concentration values of $O_2$ and $CO_2$ in endometrial fluid at a given time point.

In a preferred embodiment, the reference value is the concentration value of dissolved gas in endometrial fluid for a given female subject at a given time point prior to the start of the window of implantation in said female subject.

In some embodiments, the reference value refers to the value resulting from pooling concentration values of dissolved gas in endometrial fluid obtained from normal or healthy female subjects prior to the start of the window of implantation. In some embodiments, the reference value refers to the value resulting from pooling concentration values of dissolved gas in endometrial fluid in female subjects which are undergoing assisted reproduction procedures and/or IVF prior to the start of the window of implantation.

The term "reference value" may refer to series of concentration values of dissolved gas in endometrial fluid obtained at different, consecutive time points. A "reference value", as used herein, may refer to a concentration value of dissolved gas in endometrial fluid obtained from a different subject than the subject undergoing a determination of endometrial receptivity or a group of subjects, preferably two or more subjects, known to have a known physiological or clinical status. The suitable reference concentration value of dissolved gas in endometrial fluid can be determined by measuring the concentration of dissolved gases in several suitable subjects at a given time point, and such reference levels can be adjusted to specific subject populations or clinical settings.

Typically, the reference value can be a threshold value. Typically, a "threshold value" can be determined experimentally, empirically, or theoretically. A threshold value can also be arbitrarily selected based upon the existing experimental and/or clinical conditions, as would be recognized by a person of ordinary skill in the art. The threshold value has to be determined in order to obtain the optimal sensitivity and specificity according to the function of the test and the benefit/risk balance (clinical consequences of false positive and false negative). Typically, the optimal sensitivity and specificity (and so the threshold value) can be determined using a Receiver Operating Characteristic (ROC) curve based on experimental data. In one embodiment of the present invention, the threshold value is derived from the concentration value of dissolved gas in endometrial fluid (or ratio, or score) determined in one or more female subjects at a given time point with a high potential for pregnancy outcome. In a particular embodiment, the reference value or threshold value is selected from the group consisting of 30 Torr (4.00 KPa), 35 Torr (4.67 KPa) and 40 Torr (5.33 KPa).

In a preferred embodiment, the reference value is the concentration value of dissolved $O_2$ and/or $CO_2$ in endometrial fluid in said female subject measured at least once in the period of time comprised between 2 days before the start of ovulation and day 2 or 3 of the luteal phase in natural cycles, or between 2 days before the start of ovulation and day 1-2 after progesterone administration in artificial cycles.

The term "at least once" includes once, twice, three times, four times, five times or more times.

The term "ovulation" is the stage of the menstrual cycle at which an egg is released from the ovary, egg which may or may not be fertilized by sperm. If fertilized, the egg may travel to the uterus and implant to develop into a pregnancy. If left unfertilized, the egg disintegrates and the uterine lining is shed during the period. Ovulation typically occurs around day 14 of a 28-day of a normal menstrual cycle. However, the present invention contemplates physiological or non-physiological conditions and female subjects in which the exact timing of the stage of ovulation may highly vary. The process of ovulation begins with the body's release of follicle-stimulating hormone (FSH), typically between days 6 and 14 of the menstrual cycle which contributes to the maturation of the egg inside the ovary and triggers the production of estrogen in the follicle. Once the egg is mature, the body releases a surge of luteinizing hormone (LH), triggering the egg's release. Under normal conditions, ovulation may happen in hours 28 to 36 after the LH surge. A "luteinizing hormone (LH) surge" can be determined in urine and/or blood testing using methods known in the art. In a particular embodiment, a LH surge is defined as a serum LH concentration of at least 15 mIU/mL (milli-international units per millilitre), in a more particular embodiment, at least 20 mIU/mL.

The term "natural cycle", as used herein, refers to a cycle in the absence hormone replacement therapy. The luteal phase is the latter phase of the menstrual cycle. It begins with the formation of the corpus luteum and ends in either pregnancy or luteolysis (also luteal regression) which is structural and functional degradation of the corpus luteum (CL), which occurs at the end of the luteal phase of the menstrual cycle in the absence of pregnancy. The main hormone associated with this stage is progesterone, which is significantly higher during the luteal phase than other phases of the cycle. The luteal phase starts with the ovulation. Therefore, as used herein, day 1 of the luteal phase (LH+ 1) corresponds to the day after ovulation, day 2 of the luteal phase (LH+2) corresponds to day 2 after ovulation, day 3 of the luteal phase (LH+3) corresponds to day 3 after ovulation, and so on. Typically, the window of implantation of a natural cycle happens between LH+6 and LH+9.

The term "artificial cycle", as used herein, refers to a cycle under stimulation with hormone replacement therapy. In an artificial cycle the progesterone is administered during the luteal phase and, as used herein, P+1 corresponds to the first day after administration of progesterone, P+2 corresponds to the second day after administration of progesterone, and so on. Typically, the window of implantation of an artificial cycle happens between P+4 and P+6. Also, there are natural cycles where progesterone is administrated as a supplement. In every cycle where progesterone is administrated to develop or support the luteal phase, the window of implantation is typically happening between P+4 and P+6.

In some embodiments, the reference value is the concentration value of dissolved $O_2$ and/or $CO_2$ in endometrial fluid in another female subject or group of female subjects measured at least once in the period of time comprised between 2 days before the start of ovulation and day 2 or 3 of the luteal phase in natural cycles, or between 2 days before the start of ovulation and day 4-6 after progesterone administration in artificial cycles. Said another female subject or group of female subjects may be healthy or normal female subjects or female subjects which are undergoing assisted reproduction procedures and/or IVF.

According to the present invention, measuring the endometrial fluid dissolved gas concentration does not involve directly contacting or physically interacting with the endometrium of said female subject. The present method contemplates the measurement of dissolved gas concentration by exclusively interacting with endometrial fluid but avoiding any direct contact with endometrial tissue at the step of measuring.

As used herein, a deviation of the dissolved gas concentration value with respect to the reference value may refer to an "increase" in the concentration value with respect to the reference value.

The term "increased concentration value" or "increased concentration value of dissolved gas" as used herein in relation to the concentration value of a dissolved gas in endometrial fluid, relates to a situation where the concentration value of said dissolved gas is increased at least 1%, at least 2%, at least 3%, at least 4%, at least 5%, at least 6%, at least 7%, at least 8%, at least 9%, at least 10%, at least 20%, at least 30%, at least 40%, at least 50%, at least 60%, at least 70%, at least 80%, at least 90% or at least 100% when compared to the corresponding reference value obtained in said female subject at a previous time point.

In a preferred embodiment, an increased concentration value may be obtained in a given female subject with respect to the reference value obtained in said female subject at a previous time point. As used herein, an increased concentration value may be indicative of an implantation-synchronous endometrium in relation to an implantation-capable embryo which enters aerobic metabolism and thus requires higher oxygen concentration. The term "implantation-synchronous endometrium" refers to the state of an endometrium suitable for allowing implantation of an embryo physiologically and temporally so developed that it "can interact with the receptive endometrium so as to proceed with pregnancy.

In one embodiment, the dissolved gas concentration value may not change with respect to the reference value. As used herein, an "unchanged" dissolved gas concentration value with respect to the reference value may be indicative that the endometrium is not receptive.

As used herein, a deviation of the dissolved gas concentration value with respect to the reference value may refer to a "reduction" in the concentration value with respect to the reference value.

The term "reduced concentration value" or "reduced concentration value of dissolved gas" as used herein in relation to the concentration value of a dissolved gas in endometrial fluid, relates to a situation where the concentration value of said dissolved gas is reduced at least 1%, at least 2%, at least 3%, at least 4%, at least 5%, at least 6%, at least 7%, at least 8%, at least 9%, at least 10%, at least 20%, at least 30%, at least 40%, at least 50%, at least 60%, at least 70%, at least 80%, at least 90% or at least 100% when compared to the corresponding reference value obtained in said female subject at a previous time point.

In some embodiments, a reduced concentration value may be obtained in a given female subject with respect to the reference value obtained in said female subject at a previous time point. As used herein, a reduced concentration value may be indicative of a non-implantation-synchronous endometrium in relation to an implantation-capable embryo. The term "non-implantation-synchronous endometrium" refers to the state of an endometrium which is not suitable for allowing implantation of an embryo, so that no pregnancy takes place.

In a preferred embodiment, the term "endometrium is receptive" or "implantation-synchronous endometrium" interchangeably used herein in relation to the concentration value of dissolved $O_2$ in endometrial fluid, relates to a situation in which the concentration value of dissolved $O_2$ is increased at least 1%, at least 2%, at least 3%, at least 4%, at least 5%, at least 6%, at least 7%, at least 8%, at least 9%, at least 10%, at least 20%, at least 30%, at least 40%, at least 50%, at least 60%, at least 70%, at least 80%, at least 90% or at least 100% when compared to the corresponding reference value obtained in said female subject at a previous time point.

In some embodiments, the term "endometrium is receptive" as used herein in relation to the concentration value of dissolved $CO_2$ relates to a situation in which the concentration value of dissolved of $CO_2$ is reduced at least 1%, at least 2%, at least 3%, at least 4%, at least 5%, at least 6%, at least 7%, at least 8%, at least 9%, at least 10%, at least 20%, at least 30%, at least 40%, at least 50%, at least 60%, at least 70%, at least 80%, at least 90% or at least 100% when compared to the corresponding reference value obtained in said female subject at a previous time point.

In some embodiments, the term "endometrium is receptive" as used herein in relation to the concentration value of dissolved $O_2$ and $CO_2$ in endometrial fluid, relates to a situation in which the concentration value of dissolved $O_2$ is increased at least 1%, at least 2%, at least 3%, at least 4%, at least 5%, at least 6%, at least 7%, at least 8%, at least 9%, at least 10%, at least 20%, at least 30%, at least 40%, at least 50%, at least 60%, at least 70%, at least 80%, at least 90% or at least 100% when compared to the corresponding reference value obtained in said female subject at a previous time point, and the concentration value of dissolved of $CO_2$ is reduced at least 1%, at least 2%, at least 3%, at least 4%, at least 5%, at least 6%, at least 7%, at least 8%, at least 9%, at least 10%, at least 20%, at least 30%, at least 40%, at least 50%, at least 60%, at least 70%, at least 80%, at least 90% or at least 100% when compared to the corresponding reference value obtained in said female subject at a previous time point.

In some embodiments, the term "endometrium is not receptive" or "non-implantation-synchronous endometrium" used herein in relation to the concentration value of dissolved $O_2$ in endometrial fluid, relates to a situation in which the concentration value of dissolved $O_2$ is reduced at least 1%, at least 2%, at least 3%, at least 4%, at least 5%, at least 6%, at least 7%, at least 8%, at least 9%, at least 10%, at least 20%, at least 30%, at least 40%, at least 50%, at least 60%, at least 70%, at least 80%, at least 90% or at least 100% when compared to the corresponding reference value obtained in said female subject at a previous time point.

In some embodiments, the term "endometrium is not receptive" as used herein in relation to the concentration value of dissolved $CO_2$ in endometrial fluid relates to a situation in which the concentration value of dissolved of $CO_2$ is increased at least 1%, at least 2%, at least 3%, at least 4%, at least 5%, at least 6%, at least 7%, at least 8%, at least 9%, at least 10%, at least 20%, at least 30%, at least 40%, at least 50%, at least 60%, at least 70%, at least 80%, at least 90% or at least 100% when compared to the corresponding reference value obtained in said female subject at a previous time point.

In some embodiments, the term "endometrium is not receptive" as used herein in relation to the concentration value of dissolved $O_2$ and $CO_2$ in endometrial fluid, relates to a situation where the level of $CO_2$ is increased at least 1%, at least 2%, at least 3%, at least 4%, at least 5%, at least 6%, at least 7%, at least 8%, at least 9%, at least 10%, at least 20%, at least 30%, at least 40%, at least 50%, at least 60%, at least 70%, at least 80%, at least 90% or at least 100% when compared to the corresponding reference value obtained in said female subject at a previous time point, and the level of $O_2$ is reduced at least 1%, at least 2%, at least 3%, at least 4%, at least 5%, at least 6%, at least 7%, at least 8%, at least 9%, at least 10%, at least 20%, at least 30%, at least 40%, at least 50%, at least 60%, at least 70%, at least 80%, at least 90% or at least 100% when compared to the corresponding reference value obtained in said female subject at a previous time point.

The term "endometrium is receptive" as used herein relates to the situation in which the endometrium of the female subject shows at least 1%, at least 2%, at least 3%, at least 4%, at least 5%, at least 6%, at least 7%, at least 8%, at least 9%, at least 10%, at least 20%, at least 30%, at least 40%, at least 50%, at least 60%, at least 70%, at least 80%, at least 90%, at least 100% probabilities of acquiring a functional status that, upon transfer of a implantation-capable embryo, allows blastocyst adhesion and that will lead to pregnancy.

The term "endometrium is not receptive" as used herein relates to the situation in which the endometrium of the female subject shows at least 10%, at least 20%, at least 30%, at least 40%, at least 50%, at least 60%, at least 70%, at least 80%, at least 90%, at least 100% probabilities of not acquiring a functional status that, upon transfer of a implantation-capable embryo, allows blastocyst adhesion and that will lead to pregnancy. The term "pregnancy" is understood as the visualization of a gestational sac 4 weeks after embryo transfer.

In some embodiments, once the reference value is established, the concentration value of dissolved gas in endometrial fluid in a female subject in which the receptivity of the endometrium is to be determined can be compared with this reference value, and thus be assigned as "increased", "decreased" or "equal" in fold change with respect to the reference value obtained in said female subject at a previous time point. For example, an increase in levels above the reference value of at least 1.1-fold, at least 1.2-fold, at least 1.3-fold , at least 1.4-fold, at least 1.5-fold, 1.6-fold, at least 1.6-fold, at least 1.7-fold, at least 1.8-fold, at least 1.9-fold at least 2-fold, at least 3-fold, at least 4-fold at least 5-fold, 10-fold, 20-fold, 30-fold, 40-fold, 50-fold, 60-fold, 70-fold, 80-fold, 90-fold, 100-fold or even more compared with the reference value obtained in said female subject at a previous time point is considered as "increased" concentration value. On the other hand, a decrease in levels below the reference value of at least 0.9-fold, 0.75-fold, 0.2-fold, 0.1-fold, 0.05-fold, 0.025-fold, 0.02-fold, 0.01-fold, 0.005-fold or even less compared with reference value is considered as "decreased" concentration value obtained in said female subject at a previous time point. A concentration value can be seen as "equal" to the reference value if the concentration value differs with respect to the reference value obtained in said female subject at a previous time point is less than 1%, less than 0.5 %, less than 0.4%, less than 0.3%, less than 0.1%, less than 0.05%, or less.

As the person skilled in the art will understand, such determination does not usually seek to be correct for all (i.e., 100%) of the subjects that are going to be identified. However, the term requires that a statistically significant part of the subjects can be identified (for example, a cohort in a cohort study). The person skilled in the art can easily determine if a part is statistically significant using several well-known statistical evaluation tools, for example, determination of confidence intervals, determination of p values, Student's t-test, Mann-Whitney test, etc. The details are found in Dowdy and Wearden, Statistics for Research, John Wiley and Sons, New York 1983. The preferred confidence intervals are at least 90%, at least 95%, at least 97%, at least 98% or at least 99%. The p values are preferably 0.1, 0.05, 0.01, 0.005 or 0.0001. More preferably, at least 60%, at least 70%, at least 80% or at least 90% of the subjects of a population can be suitably identified by the method of the present invention.

In some embodiments, the endometrial fluid dissolved gas concentration is continuously measured for a period from 1 minute to 10 minutes, preferable 2 minutes to 7 minutes, more preferably from 3 minutes to 5 minutes.

In some embodiments, the endometrial fluid dissolved gas concentration is measured at least once, including at least twice, at least 3 times, at least 4 times, at least 5 times, at least 6 times, at least 7 times, or more times, in the period of time comprised between 2 days before ovulation and day 12 of the luteal phase, preferably at least once in the period of time comprised between the day of ovulation and day 10 of the luteal phase, more preferably at least once in the period of time comprised between day 2 and day 8 of the luteal phase, yet more preferably at least once in the period of time comprised between day 4 and day 7 of the luteal phase.

In some embodiments, the endometrial fluid dissolved gas concentration is measured at least once 2 days before the start of ovulation, at least once at ovulation and/or at least once at day 1, 2, 3, 4, 5, 6, 7, 8, 10 or 12 of the luteal phase.

In a particular embodiment, the measurements made from two days before the start of ovulation to two days after ovulation may be used as reference values. In this particular embodiment, detecting a deviation, in particular an increased in the concentration value of dissolved $O_2$ and/or a decreased in the concentration value of CO2, in one or more of the measurements made after ovulation compared to the measurements made before ovulation is indicative that the endometrium is receptive.

In some embodiments, the measurements made 2 days before the day of ovulation and the day of ovulation may be used as reference values.

In some embodiments, the measurements made at the day of ovulation and the day after ovulation may be used as reference values.

In some embodiments, the measurements made at the day of ovulation and 2 days after ovulation may be used as reference values.

Without wanting to be bound to any particular theory, it is considered that during the proliferative stage of the menstrual cycle oxygen diffusion from the spiral arteries to the uterine lumen decreases as a result of the thickening of the endometrium. This physiological intrauterine hypoxia protects the embryo from toxic oxygen species. However, when the morula reaches the uterus and the blastocyst begins its development, oxygen concentration in the endometrial fluid should increase to meet the metabolic demands of the embryo. Therefore, in some embodiments, the reference value is the value of dissolved $O_2$ at a concentration of physiological hypoxia in the endometrial fluid and the increase of the dissolved $O_2$ concentration value with respect to the physiological hypoxia is indicative that the endometrium of said female subject is receptive. As used herein, "physiological hypoxia" refers to a situation where the oxygen concentration in the endometrial fluid is significantly lower than the oxygen concentration in the atmosphere. Alternatively, as used herein "physiological hypoxia" refers to the situation in which the oxygen concentration within a tissue is less than 40 mmHg (5% $pO_2$) and is physiologically achieved, more particularly less than 2% $pO_2$.

In some embodiments, said female subject is undergoing an assisted reproduction procedure with a step of embryo transfer. As used herein, the phrase "assisted reproductive technique" or "assisted reproductive technology" or "assisted reproduction procedure" (ART) refers to a plurality of treatments intended to increase fertility. Non-limiting examples of ART include intrauterine insemination (IUI), intracytoplasmic sperm injection (ICSI), *in vitro* fertilization (IVF), embryo transfer into a female subject, pre-implantation genetic testing, hormone replacement therapy (HRT) cycles (e.g., exogenous administration of progesterone and estrogen), *in vitro* oocyte maturation, gamete intrafallopian transfer (GIFT), zygote intrafallopian transfer (ZIFT), surrogacy. *In vitro* fertilization (IVF) is the joining of a woman's egg and a man's sperm *in vitro.* An IVF cycle involves several steps; Step 1: Stimulation, also called super ovulation; Step 2: Egg retrieval; Step 3: Fertilization (such as IVF or ICSI); Step 4: Embryo culture; Step 5: Embryo transfer. The term "embryo transfer" as used herein, refers to the process wherein an embryo produced by any fertilization method is transferred to the uterus of a female recipient.

In some embodiments, the method of the invention further comprises, prior measuring the endometrial fluid dissolved gas concentration of said female subject, a step of detecting ovulation in said female subject by any suitable method, including methods based on the levels of luteinizing hormone in the blood or urine, ultrasound, basal body temperature (BBT) method and blood quantification of other hormones such as progesterone and estrogen. In a particular embodiment, ovulation is detected by measuring the level of luteinizing hormone (LH) and/or by ultrasound monitoring. Methods for measuring the level of luteinizing hormone (LH) and/or ultrasound monitoring are known in the art. The level of luteinizing hormone can referred to the blood level of LH or to the urine level of LH. In both cases, ovulation occurs within 12-36 hours after the LH surge has occurred. The level of LH in blood and urine can be determined by routine methods for quantification of proteins, preferably methods that comprise the use of antibodies with the capacity for binding specifically to the assayed protein, in this case LH, such as Western blot or immunoblot, ELISA (enzyme-linked immunosorbent assay), RIA (radioimmunoassay), competitive EIA (enzyme immunoassay), DAS-ELISA (double antibody sandwich ELISA), two-dimensional gel electrophoresis, capillary electrophoresis, immunocytochemical and immunohistochemical techniques, immunoturbidimetry, immunofluorescence, techniques based on the use of biochips or protein microarrays including specific antibodies or assays based on the colloidal precipitation in formats such as reagent strips and assays based on antibody-linked quantum dots. Other forms of detecting and quantifying proteins include, for instance, affinity chromatography techniques or ligand-binding assays. In a particular embodiment, the level of LH in the urine can be determine by means of a lateral flow device.

Ovulation can be detected by ultrasound, which can be abdominal or transvaginal. This method is based on the monitoring of follicular growth by measuring the follicle dimensions. Ovulation generally occurs when the follicle measures about 1.8 to 2.5 cm of diameter.

In some embodiments, the method of the invention further comprises measuring a parameter selected from the group consisting of: the concentration of luteinizing hormone (LH) in urine, the concentration of progesterone, estrogen and/or vascular endothelial growth factor (VEGF) in blood, the presence of follicles and/or corpus luteum in the ovaries, the thickness and appearance of the endometrium, and the indices of resistance and pulsatility of the uterine spiral arteries. Methods for measuring the above mentioned parameters are known in the art.

In particular, the concentration of progesterone, estrogen and VEGF in blood can be determined by any suitable method for quantification of proteins in blood sample, as explained above for LH quantification.

The presence of follicles and/or corpus luteum in the ovaries can be determined by transvaginal or abdominal ultrasound. The term "follicle", as used herein, refers to a structure in the ovary that contains and nurtures the oocyte or egg. The term "corpus luteum", as used herein, refers to a mass of cells that forms in an ovary after the follicle has released the egg and is responsible for the production of progesterone during early pregnancy. Ovulation can be detected by monitoring the growth of the follicle and the formation of the corpus luteum.

The thickness and appearance of the endometrium can be analyzed by ultrasound.

The resistance index (RI) and pulsatility index (PI) of the uterine and spiral arteries can be determined by transvaginal Doppler ultrasound. The term "pulsatility index, also known as the Gosling index, is a calculated flow parameter in ultrasound, derived from the maximum, minimum, and mean Doppler frequency shifts during a defined cardiac cycle. The pulsatility index is calculated by one of the following equations:

$$PI=(V_{max}-V_{min})/(V_{mean})$$

PI= (peal systolic velocity-minimal diastolic velocity)/(mean velocity) The term "resistance index", also known as "Pourcelot index", refers to a calculated flow parameter in ultrasound, derived from the maximum, minimum, and mean Doppler frequency shifts during a defined cardiac cycle. The resistance index is calculated with the formula: RI= (PSV-EDV)/PSV, where PSV means peak systolic velocity and EDV means end-diastolic velocity.

In some embodiments, the endometrial fluid dissolved gas concentration is measured in the uterine fundus and/or in the cervical canal of said female subject, preferably in the cervical canal of the uterus.

The term "uterine fundus", as used herein, refers to the broad curved upper area of the uterus in which the fallopian tubes connect to the uterus. In some embodiments, the dissolved gas concentration measurements are performed at least 1 cm away from the uterine fundus.

The term "cervical canal", as used herein, refers to the lower part of the uterus extending downward from the isthmus until it opens into the vagina. In some embodiments, the step of measuring the endometrial fluid dissolved gas concentration is performed by a sensor capable of measuring the endometrial fluid dissolved gas concentration, which sensor does not contact the endometrium of said female subject. Non-limiting examples of sensors capable of measuring the endometrial fluid dissolved gas concentration are commercially available sensors such as PreSens Oxygen Microsensors, Neotrend, Paratrend, Neurotrend, NEUROVENT-PTO and Licox.

In some embodiments, the step of measuring the endometrial fluid dissolved gas concentration is performed *in vitro* in a sample of endometrial fluid. The term *"in vitro",* as used herein, refers to the fact that the method is not carried out on the body of a human or animal subject, but rather on cells or fluids isolated from said subject or in a test tube. Methods for obtaining samples endometrial fluid are well known to those skilled in the art, for example, by aspiration of fluid comprised in the uterine cavity. In order to carry out this particular embodiment of the method of the invention, the contact between the sample of endometrial fluid and atmosphere should be avoided so that the dissolved gas content in the sample of endometrial fluid is not altered.

In another particular embodiment, the sensor for measuring the dissolved $O_2$ or CO2 concentration is located inside the catheter and a sample of endometrial fluid is aspirated inside the catheter, so that the measure is performed inside the catheter while it is in the uterus, preferably in the cervical canal.

### 2. *Method for selecting a female subject undergoing an ART procedure* as a *candidate to receive an embryo*

The authors have found that the measurement of the dissolved gas concentration in endometrial fluid of a female subject allows for the selection of a female subject undergoing an ART procedure as a candidate to receive an embryo to be performed on the same day as the measurement of dissolved gas. The identification of a female subject whose endometrium is receptive or not receptive is the basis for a clinical decision in certain steps of assisted reproduction approaches. Such clinical decision may involve selecting an implantation-capable embryo and transfer said embryo into the uterus of a female subject, if the endometrium is receptive. On the contrary, if the endometrium is not (yet) receptive, the subject is asked to return on the next day for another measurement. Finally, if it is post-receptive, the selection of an implantation-capable is dispensed with and the embryo transfer is no longer scheduled. The present method provides with a basis for a reliable clinical decision in short time, even within minutes. The schedule of subsequent steps in assisted reproduction procedures can be assessed much faster than with conventional methods. In addition, measuring the dissolved gas concentration in endometrial fluid of a female subject for assessing endometrial receptivity at a given time point does not require collection of endometrial biopsies. The measurements may be performed with the standard equipment used for *in vitro* fertilization procedures, more specifically, for the step of embryo transfer into the uterus of a female subject. The measurement approach according to the invention represents a very mild procedure which is generally not associated with pain or tissue damage in the female subject. Accordingly, the step of measuring the dissolved gas concentration is compatible with performing subsequently, as short as within few hours from the measuring step, the embryo transfer in the same female subject if the endometrium of said female subject is determined as being receptive with the first method of the invention.

Thus, in a further aspect the invention relates to a method for selecting a female subject undergoing an assisted reproduction procedure as a candidate to receive an embryo (second method of the invention), the method comprising the steps of:

i) determining whether the endometrium of said female subject is receptive following the method steps according to the first method of the invention, and if said endometrium is receptive,
ii) selecting said female subject as a candidate to receive an embryo in an embryo transfer procedure to be performed as soon as on the same day as step i).

As used herein, the term "procedure to be performed as soon as" refers to the possible earliest time point at which a given procedure may be performed without limitation to be performed at a later time point with respect to said earliest time point. The term "as soon as on the same day as step i)" is understood such that, according to the invention, the earliest time point for an embryo transfer procedure may be the day at which the determination of endometrial receptivity is carried out, however, without limitation to be performed at a later time point, for example, 1 day after determination of endometrial receptivity, 2 days after determination of endometrial receptivity, and so on.

In a first step of the second method of the invention, the concentration of dissolved gas in endometrial fluid is measured and the receptivity of the endometrium of a female subject is determined as described previously in the context of the first method of the invention. Methods to measure the concentration of dissolved gas in endometrial fluid described previously in the context of the first method of the invention, are applicable to the second method of the invention.

Embodiments disclosed in the context of the first method of the invention are also contemplated for the second method of the invention.

### 3. *Method for achieving pregnancy in a female subject*

The authors have found that measuring the dissolved gas concentration in endometrial fluid of a female subject makes possible achieving pregnancy in a female subject, in particular, a female subject undergoing assisted reproduction procedures. The expression "determining the endometrial receptivity" as used herein relates to the assessment of a physiological or clinical status of the endometrium with respect to the pregnancy outcome (e.g. likelihood of pregnancy or implantation-capable embryo survival). As used herein the term "pregnancy" is understood as the visualization of a gestational sac 4 week after embryo transfer. The term pregnancy may also be understood as the result of the final result of a fertilization event.

The term "method for achieving pregnancy", as used herein, refers to any kind of measure either prophylactic or therapeutic, including means of any class, such as hygienic means, pharmacological means, surgical means or physical means, with the purpose of promoting pregnancy in a female subject.

Thus, in a further aspect the invention relates to a method for achieving pregnancy in a female subject (third method of the invention), the method comprising the steps of:

i) measuring the endometrial fluid dissolved gas concentration of said female subject, thereby obtaining a dissolved gas concentration value, wherein the dissolved gas is selected from the group consisting of $O_2$ and $CO_2$,
ii) comparing said dissolved gas concentration value with a reference value, wherein an increase of the concentration value of dissolved $O_2$ and/or a decrease in the concentration value of dissolved $CO_2$ with respect to the reference value is indicative that the endometrium of said female subject is receptive, and
if the dissolved gas concentration value deviates from said reference value,
iii) transferring an embryo into the receptive endometrium of said female subject, and
iv) allowing the progression of pregnancy.

Alternatively, the invention relates to a method for treating infertility in a female subject, comprising:

i) measuring the endometrial fluid dissolved gas concentration of said female subject, thereby obtaining a dissolved gas concentration value, wherein the dissolved gas is selected from the group consisting of $O_2$ and $CO_2$,
ii) comparing said dissolved gas concentration value with a reference value, and
if there is an increase in the concentration value of dissolved $O_2$ and/or a decrease in the concentration value of $CO_2$ compared to a reference value,
iii) treating infertility by transferring an embryo into the receptive endometrium of said female subject and allowing implantation of the embryo.

In a first step of the third method of the invention, the concentration of dissolved gas in endometrial fluid is measured and the receptivity of the endometrium of a female subject is determined as described previously in the context of the first method of the invention. Methods to measure the concentration of dissolved gas in endometrial fluid described previously in the context of the first method of the invention, are applicable to the third method of the invention.

Embodiments disclosed in the context of the first method of the invention are also contemplated for the third method of the invention.

The invention is detailed below by means of the following examples which are merely illustrative and by no means limiting the scope of the invention.

**EXAMPLES**

In the absence of a reliable method of WOI detection, the multifactorial complexity of endometrial receptivity and the lack of known biomarkers, biophysical parameters such as oxygen tension and carbon dioxide tension are investigated as indicators of a healthy and properly functioning endometrium. These parameters have a dynamic behavior inside the uterus and, importantly, they have to be synchronized with the needs of the embryo at different stages of its development. In this sense, although the oxygen requirement is low when the embryo resides in the oviduct, up to the morula stage, the oxygen requirement increases dramatically when it becomes a blastocyst, and the use of glucose as an energy source to ensure its biosynthetic activity is increased. Nutrient availability, oxygen concentration and the redox state of the blastocyst regulate its metabolism and implantation potential. Considering that the oxygenation of the embryo depends on the oxygen dissolved within the oviduct and in the endometrial fluid, changes in oxygen concentration within the uterus have to be synchronized with the metabolic development of the embryo. Thus, during the proliferative stage of the menstrual cycle, the endometrium thickens and the diffusion of oxygen from the spiral arteries into the uterine lumen becomes more difficult. This physiological intrauterine hypoxia protects the embryo from toxic oxygen species and if not established, as in the case of thin endometrium, the achievement of pregnancy is hindered. However, when the morula reaches the interior of the uterus and the blastocyst begins its development, the oxygen concentration in the endometrial fluid has to be increased, since low oxygen concentrations would inhibit mitochondrial activity and glucose metabolism, having a detrimental impact on blastocyst development and implantation. In other words, intrauterine oxygen pressure must be synchronized with the metabolic requirements of the embryo according to its stage of development: hypoxia during the cellular phases and higher oxygen concentration from the blastocyst stage and during implantation.

Previous studies showed that from ovulation and during the luteal phase there is significant growth and coiling of spiral arteries, maturation of the subepithelial capillary plexus and an increase in the density of branch points of blood vessels, coinciding with the production of progesterone and a significant increase in the pro-angiogenic factor VEGF and its receptors. This process would facilitate the diffusion of oxygen from the blood vessels into the endometrial fluid and could result in an increase in intrauterine oxygen concentration. Studies in humans suggest that oxygen increases and carbon dioxide decreases in the luteal phase with respect to the proliferative phase, whereas no difference between measurements made in the uterine fundus or cervical canal are observed. Further, the balance of these gases appear to change in some uterine pathologies such as myoma, cervical carcinoma or endometrial carcinoma. Oxygen homeostasis is a complex process that reflects the proper functioning of a biological system as a whole. It can be altered by infection, inflammation, cancer, or by a defect in angiogenesis or hormonal regulation. It is also known that i) the embryo *in vivo* consumes the oxygen dissolved in the endometrial fluid, ii) the concentration of oxygen in this fluid depends on the diffusion of the gas from the blood vessels to the lumen of the uterus, iii) this diffusion depends on the distance between the vessels and the lumen and, therefore, on the thickness of the endometrium and the development of these blood vessels.

However, there is no study relating the change in trend of the dissolved oxygen concentration in endometrial fluid as an indication of the beginning of endometrial receptivity. Accordingly, the present invention provides a method for detecting the window of implantation in a female subject on the basis of a change in trend of the dissolved oxygen concentration in endometrial fluid, whereas an increasing trend with respect to a previously obtained reference value in the same subject is associated with the occurrence of endometrial receptivity. In other words, the inventors have found that the oxygen measurement in endometrial fluid represents a reliable approach to assess whether the endometrium has developed properly and whether it is synchronized and receptive to the blastocyst at the time of embryo transfer in ART procedures, with the aim of achieving improved implantation rates.

Importantly, the method of the invention is advantageous over the prior art because it does not rely on the analysis of biopsy samples and hence it does not affect the integrity of the endometrial tissue, preserving a suitable environment for a subsequent embryo implantation. The method of the invention is advantageous because it represents a method for detecting WOI, which can be performed in the same cycle in which the embryo is transferred and which is compatible with natural cycles, allowing in turn the personalization of ART protocols focused on increasing the success of the treatment, facilitating natural cycles, reducing the number of transfers necessary to achieve pregnancy, and thereby reducing the impact on the mental and physical health of the families and the economic cost to the health system.

The method of the invention is aimed at detecting endometrial receptivity and synchrony through the non-invasive measurement of intrauterine oxygen pressure in endometrial fluid, for example, by optical means. Oxygen optical sensors are safe, accurate, and have been used in different clinical applications such as continuous blood gas monitoring of sedated patients and critically ill neonates and brain oxygen monitoring in patients with acute brain injury. Measurement of dissolved oxygen concentration by means of oxygen optical sensors may successfully be carried out on the day of embryo transfer using a transfer catheter (such catheters as used for embryo transfer in IVF) for placing the optical microsensor in the uterine fundus in contact with the endometrial fluid, however, without contacting the endometrium.

## Materials and Methods

### Subject population

5 young (18-35 years) healthy female volunteers with BMI < 30, regular cycles, non-smokers and normal uterine morphology. Exclusion criteria:

- Medical pathology: Insulin-dependent diabetes mellitus, Cushing's syndrome, thyroid dysfunction, hepatic and/or renal failure, pathology contraindicating ovarian stimulation and/or gestation and antiphospholipid syndrome.
- Uterine pathology (endometriosis, cancer, malformations, polyps, myomas).
- Smoker or drugs consumption
- Treatment with oral contraceptives or IUD in the last 3 months.

### Experimental design

A sterile optical oxygen sensor previously used in the detection of intrauterine oxygen concentration is used (Ottosen et al. "Observations on intrauterine oxygen tension measured by fiber-optic microsensors" Reproductive biomedicine online vol. 13,3 (2006): 380-5). The method in Ottosen *et al.* for the detection of intrauterine oxygen in women uses a fiber optic microsensor and a transfer catheter for its introduction into the uterus. The kind of oxygen sensor used is also part of several approved medical devices for the detection of oxygen in blood and brain: Neurotrend, Neotrend, Paratrend, NEUROVENT-PTO, Raumedic neuro-icu (https://www.raumedic.com/neuromonitoring/neuro-icu).

Measuring characteristics are determined by the manufacturer. Measuring range of the sensor is 0-500% air saturation (air sat.). Due to the measuring principle of the sensor, resolution varies with partial pressure. Resolution at 1, 30, 100, 250% air sat. was 0.05, 0.1, 0.5 and 1.7% air sat. respectively. Resolution in the uterine cavity is thus better than 0.1% air sat. Accuracy is within $\pm$ 1% air sat. The 0-90% response time is <5-10 s. The sensor shows no cross-sensitivity for carbon dioxide or any ionic species (www.presense.de).

The intrauterine oxygen tension is measured with a specially devised type of fibre-optic microsensor, made of thin flexible insulated optical fibres with an outer diameter of 0.9 mm. These miniaturized implantable probes are suitable for various applications, e.g. insertion into the blood circuit or tissue, and yield online real time oxygen measurements. The detection principle is based on quenching by oxygen of the fluorescent dye (a fluorophore) immobilized at the tip of the fibre and subsequently coated with an optical insulation to avoid interference from intrinsic fluorescence (PreSens, Regensburg, Germany, www.presens.de). The decrease of the fluorescent quantum yield of the fluorophore [ruthenium(II)-tris-(4,7-diphenyl-1,10-phenanthroline], as well as the fluorescent decay time is proportional to the concentration of oxygen bound to the matrix in which the dye is immobilized. The fluorescent decay time is recorded with an oxygen meter OXY-1-ST (www.presense.de), which is connected to a laptop computer. In detail, a catheter-coupling device containing a rotative Male Luer Lock ending is fixed to the sensor, in a position allowing less than 1mm sensor tip exposure into the endometrial fluid when the Luer Lock is rotated. The guiding cannula of the Embryo Transfer Catheter Set form Labotect, containing a Luer connection at the proximal end is used to enter the uterine cavity under ultrasound assistance. When the end of the cannula is 1 cm away from the uterine fundus, the microsensor is introduced through it and the Luer lock is rotated, leaving the microsensor tip "swimming" in endometrial fluid, but never in direct contact with endometrial tissue. The rounded distal end of the labotect guiding cannula was found to be particularly helpful in this sense. Less than 1 mm of the sensor tip is extended shortly before starting the measurements and retracted before removing the sensor from the uterine cavity.

This precise extension of the sensor tip achieved by fixing the catheter coupling device to the sensor, avoids exposing the sensor by hand to the uterine lumen. Notably, the controlled extension of the sensor by less than 1 mm into the endometrial fluid is advantageous because it substantially increases the accuracy of measurements with respect to known methods. This procedure guarantees safety and results' reproducibility: ensures that the sensor tip doesn't break or damage, does not contact the endometrial tissue getting exposed only to the uterine fluid and that the measurements are always carried out in the same position in the uterine fundus.

When the measurements are carried out, the catheter is retracted from the uterus down to the cervical canal to measure oxygen concentration in the cervix.

These determinations are performed once in the early follicular phase (between days 3-5 of the menstrual cycle) and once every 48h, between days 14 and 26 of the same cycle. The concentration of dissolved oxygen in endometrial fluid is measured in the uterine fundus and cervical canal. The values obtained are compared with the following parameters: presence of follicles and corpus luteum, endometrial thickness and appearance, resistance and pulsatility indices, LH concentration in urine and Progesterone and Estrogen in blood.

*Experimental schedule*

At home:

- Urine LH detection every day from 10 to 17 of the cycle using lateral flow strips (LH-lateral flow strips).

At the IVF clinic:

- Ultrasound monitoring of follicle and corpus luteum appearance, as well as endometrial thickness and appearance. In addition, the resistance and pulsatility indexes are determined by Doppler ultrasound. This monitoring is done one day between days 3 and 5 of the cycle and on alternate days between days 14 to 26 of the menstrual cycle at the IVF clinic.
- Oxygen measurement in endometrial fluid are performed according to the method of the invention using micrometer optical fibers that inserted into the intrauterine cavity using a transfer catheter. The insertion is monitored by abdominal ultrasound. The measurement is performed for 5-10 minutes in the uterine fundus and another 5-10 minutes in the cervical canal. These measurements are carried out one day between days 3 and 5 of the cycle and on alternate days between days 14 to 26 of the menstrual cycle using the IVF consultation.

In the nurse's office:

- Blood draws are performed after fasting for 8 hours on one day between days 3 and 5 of the cycle and on alternate days between day 14 and day 26 of the menstrual cycle A.
- Determination of blood oxygen saturation is performed one day between days 3 and 5 of the cycle and on alternate days between days 14 to 26 of the menstrual cycle using a Pulsoximeter.

*Results* One-way ANOVA followed by Turkey's multiple comparisons test was performed using GraphPad Prism version 9.3.1 for macOS, GraphPad Software, San Diego, California USA, www.graphpad.com".

This study was performed at the Assisted Reproductive Unit of Vall d'Hebron Hospital in Barcelona. As a result, the first-human data describing the intrauterine oxygen pattern throughout the luteal phase of fertile women's menstrual cycle have been obtained. The $pO_2$ patterns indicate WOI occurrence in 80% of the participants (Figure 4: A, B), described by a peak of the dissolved oxygen concentration. The descriptive analytics of the data represented in Figure 4 is included in Tables 1, 2 and 3.

**Table 1.** Descriptive analytics: Data represented in Figure 4A

| | LH1 | LH2 | LH3 | LH4 | LH5 | LH6 | LH7 | LH8 | LH9 | LH10 |
|---|---|---|---|---|---|---|---|---|---|---|
| Number of values | 224 | 180 | 359 | 180 | 360 | 180 | 328 | 180 | 352 | 180 |
| 25% Percentile | 0.5913 | 2.859 | 18.86 | 17.53 | 25.24 | 41.97 | 37.75 | 30.86 | 9.962 | 2.758 |
| Median | 1.643 | 4.767 | 21.66 | 19.52 | 29.17 | 44.41 | 42.47 | 32.38 | 21.94 | 5.102 |
| 75% Percentile | 22.86 | 7.53 | 24.72 | 21.79 | 35.2 | 45.15 | 46.2 | 33.29 | 32.86 | 7.972 |
| 95% CI of median | | | | | | | | | | |
| Actual confidence level | 96.19% | 95.61% | 95.52% | 95.61% | 96.03% | 95.61% | 95.91% | 95.61% | 95.16% | 95.61% |
| Lower confidence limit | 1.024 | 4.102 | 20.8 | 19.03 | 28.09 | 43.51 | 41.8 | 32.03 | 19.42 | 4.162 |
| Upper confidence limit | 3.038 | 5.555 | 22.41 | 20.01 | 30.13 | 44.81 | 43.55 | 32.63 | 26.89 | 6.199 |
| | | | | | | | | | | |
| Mean | 10.18 | 5.559 | 21.49 | 19.39 | 30.82 | 43.75 | 41.86 | 32.2 | 22.13 | 5.518 |
| Std. Deviation | 12.13 | 3.396 | 4.886 | 3.355 | 7.6 | 1.999 | 4.865 | 1.641 | 12.4 | 3.137 |
| Std. Error of Mean | 0.8105 | 0.2531 | 0.2579 | 0.25 | 0.4006 | 0.149 | 0.2686 | 0.1223 | 0.661 | 0.2338 |
| | | | | | | | | | | |
| Lower 95% CI of mean | 8.583 | 5.06 | 20.99 | 18.89 | 30.04 | 43.45 | 41.33 | 31.96 | 20.83 | 5.057 |
| Upper 95% CI of mean | 11.78 | 6.059 | 22 | 19.88 | 31.61 | 44.04 | 42.39 | 32.44 | 23.43 | 5.98 |

**Table 2.** Descriptive analytics: Data represented in Figure 4B

|  | LH1 | LH3 | LH5 | LH7 | LH9 | LH11 |
|---|---|---|---|---|---|---|
| **Number of values** | 180 | 152 | 139 | 180 | 180 | 180 |
|  |  |  |  |  |  |  |
| **25% Percentile** | 5.245 | 15.63 | 43.92 | 6.197 | 13.24 | 9.233 |
| **Median** | **6.834** | **16.06** | **44.52** | **8.96** | **15.1** | **9.974** |
| **75% Percentile** | 8.537 | 16.56 | 45.25 | 10.12 | 16.9 | 10.7 |
|  |  |  |  |  |  |  |
| **95% CI of median** |  |  |  |  |  |  |
| **Actual confidence level** | 95.61% | 95.78% | 95.86% | 95.61% | 95.61% | 95.61% |
| **Lower confidence limit** | 6.594 | 15.79 | 44.34 | 8.408 | 14.42 | 9.749 |
| **Upper confidence limit** | 7.005 | 16.3 | 44.68 | 9.4 | 15.63 | 10.24 |
|  |  |  |  |  |  |  |
| **Mean** | **7.044** | **16.1** | **44.47** | **8.186** | **15.35** | **9.815** |
| **Std. Deviation** | 2.478 | 0.6419 | 0.9436 | 3.738 | 2.288 | 1.123 |
| **Std. Error of Mean** | 0.1847 | 0.05207 | 0.08004 | 0.2786 | 0.1706 | 0.08371 |
|  |  |  |  |  |  |  |
| **Lower 95% CI of mean** | 6.679 | 16 | 44.32 | 7.636 | 15.01 | 9.65 |
| **Upper 95% CI of mean** | 7.408 | 16.21 | 44.63 | 8.736 | 15.68 | 9.981 |

**Table 3.** Descriptive analytics: Data represented in Figure 4A

|  | LH2 | LH4 | LH6 | LH9 | LH11 | LH13 |
|---|---|---|---|---|---|---|
| Number of values | 180 | 167 | 162 | 176 | 168 | 133 |
|  |  |  |  |  |  |  |
| 25% Percentile | 20.61 | 9.377 | 1.426 | 2.9 | 11.48 | 0.803 |
| **Median** | **22.57** | **12.92** | **2.433** | **4.67** | **12.74** | **0.805** |
| 75% Percentile | 24.62 | 16.04 | 4.876 | 6.323 | 14.88 | 0.812 |
|  |  |  |  |  |  |  |
| 95% CI of median |  |  |  |  |  |  |
| Actual confidence level | 95.61% | 95.61% | 95.08% | 95.85% | 96.31% | 96.30% |
| Lower confidence limit | 21.91 | 11.81 | 2.008 | 4.083 | 12.26 | 0.804 |
| Upper confidence limit | 23.02 | 13.66 | 3.052 | 5.101 | 14.09 | 0.807 |
|  |  |  |  |  |  |  |
| **Mean** | **22.79** | **13.81** | **3.147** | **4.58** | **12.97** | **0.807** |
| Std. Deviation | 2.977 | 5.955 | 2.058 | 2.212 | 3.178 | 0.00794 |
| Std. Error of Mean | 0.2219 | 0.4608 | 0.1617 | 0.1667 | 0.2452 | 0.000689 |
|  |  |  |  |  |  |  |
| Lower 95% CI of mean | 22.35 | 12.9 | 2.827 | 4.25 | 12.49 | 0.8056 |
| Upper 95% CI of mean | 23.22 | 14.72 | 3.466 | 4.909 | 13.45 | 0.8084 |

Near the ovulation day or shortly after ovulation occurs, physiologic hypoxia is achieved, and the dissolved oxygen pressure reaches values below 10 Torr (1,4% or 1333 Pa) at the uterine cavity fundus. Women following a standard pattern (Figure 4A) have $pO_2$ values around 20 Torr until day LH+4 and increase until 30 Torr on day LH+5. On day LH+6, the $pO_2$ reaches the maximum (40-45 Torr) and remains stable on day LH+7. Then oxygen levels start decreasing on day LH+8, reaching values around 30 Torr again. This pattern correlates with the historical data collected by anatomy pathology and gene expression, indicating that the window of implantation occurs between LH + 6 and LH + 9 in natural cycles or between P + 4 and P + 7 in hormonal replacement therapy (HRT) cycles. It has been found that the uterine environment is prepared for the embryo on day LH+6, and on day LH+8, the WOI starts closing (Figure 4A).

For successful embryo implantation and ongoing pregnancy, the cross-talk between the receptive endometrium and the embryo is critical. On one side, the endometrium secretes multiple factors, proteins, or vesicles into the uterine fluid needed for blastocyst development and trophoblast invasion. On the other, the embryo modulates the endometrium for successful implantation. Several cytokines (such as the placenta growth factor) are up-regulated in the human trophectoderm of blastocyst day 5. The receptive epithelium mounts a transcriptional response to trophoblast challenge and, when attached to the endometrial epithelium, induces an early and transient transcriptional up-regulation in the receptive epithelium.

Therefore, the $pO_2$ level decreasing in LH+8 should be in response to the absence of an embryo in the uterine cavity, provoking the signalling of WOI closing. It is known that invasive placentation and pregnancy occur in the presence of a viable embryo. However, in its absence, the endometrium undergoes breakdown, remodelling, and regeneration.

Figure 4B shows a $pO_2$ profile of a woman having a shortened and advanced WOI. As noted, physiological hypoxia was achieved at the beginning of the luteal phase. However, on day LH+5, the dissolved oxygen level is already in the 40-45 Torr range, corresponding to the $pO_2$ values of LH+6 and LH+7 days in a standard profile (Figure 4A). In addition, the oxygen concentration decreased rapidly, reaching $pO_2$ values below 10 Torr on day LH+7. Therefore, the WOI would be already closed if embryo transfer were performed on LH+7 to this woman. In addition, 20% of participants showed no increase in $pO_2$, so a disrupted WOI is suspected. Women displaying this $pO_2$ profile should undergo an in-depth evaluation of endometrial function.

In addition, at least two other parameters can be combined with the $pO_2$-WOI detection to treat infertility and recommend embryo transfer: blood progesterone level and endometrial thickness.

Low blood progesterone levels on the day of embryo transfer are linked to poorer outcomes after fresh or frozen embryo transfer, and progesterone levels between 10 and 20 ng/mL on the day of embryo transfer have been shown to be optimal. Therefore, individualized progesterone support after embryo transfer could benefit these patients. Also, thin endometrium ($\leq$ 7mm) in the late follicular phase may be associated with failed implantation and lower live birth rates.

The intrauterine oxygen measurement used alone or combined with progesterone level and/or endometrial thickness prior to embryo transfer will allow recommendations for infertility treatment. Options could be to proceed with the embryo transfer, supplement progesterone in an individualized manner, treat thin endometrium, get an in-depth analysis of endometrial function, or suspend the transfer for upcoming natural or artificial IVF cycles.

## Claims

1. Method for determination of endometrial receptivity in a female subject, the method comprising the steps of:

    i) measuring the endometrial fluid dissolved gas concentration of said female subject, thereby obtaining a dissolved gas concentration value, wherein the dissolved gas is selected from the group consisting of $O_2$ and $CO_2$,

    ii) comparing said dissolved gas concentration value with a reference value, wherein an increase of the concentration value of dissolved $O_2$ and/or a decrease of the concentration value of dissolved $CO_2$ with respect to the reference value is indicative that the endometrium of said female subject is receptive.

2. Method according to claim 1, wherein the step of measuring the endometrial fluid dissolved gas concentration does not involve contacting the endometrium of said female subject.

3. Method according to any one of claims 1 or 2, wherein a deviation of the dissolved gas concentration value with respect to the reference value is indicative of an implantation-synchronous endometrium in relation to an implantation-capable embryo.

4. Method according to any one of claims 1 to 3, wherein the reference value is the concentration value of dissolved $O_2$ or $CO_2$ in endometrial fluid in said female subject measured at least once in the period of time comprised between 2 days before the start of ovulation and day 2 or 3 of the luteal phase.

5. Method according to any one of claims 1 to 4, wherein the reference value is the concentration value of dissolved $O_2$ or $CO_2$ in endometrial fluid in another female subject or group of female subjects measured at least once in the period of time comprised between 2 days before the start of ovulation and day 2 or 3 of the luteal phase.

6. Method according to any one of claims 1 to 5, wherein the endometrial fluid dissolved gas concentration is measured at least once in the period of time comprised between 2 days before ovulation and day 12 of the luteal phase, preferably at least once in the period of time comprised between the day of ovulation and day 10 of the luteal phase, more preferably at least once in the period of time comprised between day 2 and day 8 of the luteal phase, yet more preferably at least once in the period of time comprised between day 3 and day 6 of the luteal phase.

7. Method according to any one of claims 1 to 6, wherein the endometrial fluid dissolved gas concentration is measured at least once 2 days before the start of ovulation, at least once at ovulation and/or at least once at day 2, 4, 6, 8, 10 or 12 of the luteal phase.

8. Method according to any one of claims 1 to 7, wherein the reference value is the value of dissolved $O_2$ at a concentration of physiological hypoxia in the endometrial fluid and the increase of the dissolved $O_2$ concentration value with respect to the physiological hypoxia is indicative that the endometrium of said female subject is receptive.

9. Method according to any one of claims 1 to 8, wherein said female subject is undergoing an assisted reproduction procedure with a step of embryo transfer.

10. Method according to any one of claims 1 to 9, the method further comprising, prior step i), a step of detecting ovulation in said female subject by measuring the level of luteinizing hormone (LH) and/or by ultrasound monitoring.

11. Method according to any one of claims 1 to 10, the method further comprising measuring a parameter selected from the group consisting of: the concentration of LH in urine, the concentration of progesterone, estrogen and/or VEGF (vascular endothelial growth factor) in blood, the presence of follicles and/or corpus luteum in the ovaries, the thickness and appearance of the endometrium, and the indices of resistance and pulsatility of the uterine spiral arteries.

12. Method according to any one of claims 1 to 11, wherein the endometrial fluid dissolved gas concentration is measured in the uterine fundus and/or in the cervical canal of said female subject.

13. Method according to any one of claims 1 to 12, wherein the step of measuring the endometrial fluid dissolved gas concentration is performed by a sensor capable of measuring the endometrial fluid dissolved gas concentration, which sensor does not contact the endometrium of said female subject.

14. Method according to any one of claims 1 to 13, wherein the step of measuring the endometrial fluid dissolved gas concentration is performed in vitro in a sample of endometrial fluid.

15. Method for selecting a female subject undergoing *assisted reproduction techniques (ART)* as a candidate to receive an embryo, the method comprising the steps of:

   i) determining whether the endometrium of said female subject is receptive following the method steps according to any of claims 1 to 14, and if said endometrium is receptive,
   ii) selecting said female subject as a candidate to receive an embryo in an embryo transfer procedure to be performed as soon as on the same day as step i).

**Patentansprüche**

1. Verfahren zur Bestimmung der Empfänglichkeit des Endometriums bei einer weiblichen Person, wobei das Verfahren die folgenden Schritte umfasst:

   i) Messen der Konzentration gelöster Gase in der Endometriumflüssigkeit der weiblichen Person, wodurch ein Wert für die Konzentration gelöster Gase erhalten wird, wobei die gelösten Gase aus der Gruppe ausgewählt sind, die aus $O_2$ und $CO_2$ besteht,
   ii) Vergleichen des Wertes der Konzentration gelöster Gase mit einem Referenzwert, wobei ein Anstieg des Wertes der Konzentration von gelöstem $O_2$ und/oder ein Abfall des Wertes der Konzentration von gelöstem $CO_2$ in Bezug auf den Referenzwert darauf hinweist, dass das Endometrium der weiblichen Person empfänglich ist.

2. Verfahren nach Anspruch 1, wobei der Schritt des Messens der Konzentration von gelöstem Gas in der Endometriumflüssigkeit keine Kontaktaufnahme mit dem Endometrium der weiblichen Person beinhaltet.

3. Verfahren nach irgendeinem der Ansprüche 1 oder 2, wobei eine Abweichung des Wertes der gelösten Gaskonzentration in Bezug auf den Referenzwert auf ein implantationssynchrones Endometrium in Bezug auf einen implantationsfähigen Embryo hinweist.

4. Verfahren nach irgendeinem der Ansprüche 1 bis 3, wobei der Referenzwert der Konzentrationswert von gelöstem $O_2$ oder $CO_2$ in der Endometriumflüssigkeit bei der genannten weiblichen Person ist, der mindestens einmal in dem Zeitraum umfassend zwischen 2 Tagen vor Beginn des Eisprungs und dem 2. oder 3. Tag der Lutealphase gemessen wird.

5. Verfahren nach irgendeinem der Ansprüche 1 bis 4, wobei der Referenzwert der Konzentrationswert von gelöstem $O_2$ oder $CO_2$ in der Endometriumflüssigkeit bei einer anderen weiblichen Person oder einer Gruppe weiblicher Personen ist, gemessen mindestens einmal in dem Zeitraum umfassend zwischen 2 Tagen vor Beginn des Eisprungs und dem 2. oder 3. Tag der Lutealphase.

6. Verfahren nach irgendeinem der Ansprüche 1 bis 5, wobei die Konzentration von gelöstem Gas in der Endometriumflüssigkeit mindestens einmal in dem Zeitraum umfassend zwischen 2 Tagen vor dem Eisprung und dem 12. Tag der Lutealphase gemessen wird, bevorzugt mindestens einmal in dem Zeitraum umfassend zwischen dem Tag des Eisprungs und dem 10. Tag der Lutealphase gemessen wird, mehr bevorzugt mindestens einmal in dem Zeitraum umfassend zwischen dem 2. und 8. Tag der Lutealphase gemessen wird, noch bevorzugt mindestens einmal in dem Zeitraum umfassend zwischen dem 3. und 6. Tag der Lutealphase gemessen wird.

7. Verfahren nach irgendeinem der Ansprüche 1 bis 6, wobei die Konzentration von gelöstem Gas in der Endometriumflüssigkeit mindestens einmal 2 Tage vor Beginn des Eisprungs, mindestens einmal beim Eisprung und/oder mindestens einmal am Tag 2, 4, 6, 8, 10 oder 12 der Lutealphase gemessen wird.

8. Verfahren nach irgendeinem der Ansprüche 1 bis 7, wobei der Referenzwert der Wert von gelöstem $O_2$ bei einer Konzentration von physiologischer Hypoxie in der Endometriumflüssigkeit ist und der Anstieg des Wertes der gelösten $O_2$-Konzentration in Bezug auf die physiologische Hypoxie darauf hinweist, dass das Endometrium der weiblichen Person empfänglich ist.

9. Verfahren nach irgendeinem der Ansprüche 1 bis 8, wobei die weibliche Person sich einem Verfahren der assistierten Reproduktion mit einem Schritt des Embryotransfers unterzieht.

10. Verfahren nach irgendeinem der Ansprüche 1 bis 9, wobei das Verfahren vor Schritt i) einen Schritt zum Nachweis des Eisprungs bei der weiblichen Person durch Messung des Spiegels des luteinisierenden Hormons (LH) und/oder durch Ultraschallüberwachung umfasst.

11. Verfahren nach irgendeinem der Ansprüche 1 bis 10, wobei das Verfahren ferner das Messen eines Parameters umfasst, der aus der Gruppe ausgewählt ist, die besteht aus: der Konzentration von LH im Urin, der Konzentration von Progesteron, Östrogen und/oder VEGF (vaskulärer endothelialer Wachstumsfaktor) im Blut, dem Vorhandensein von Follikeln und/oder Gelbkörper in den Eierstöcken, die Dicke und das Aussehen des Endometriums und die Indizes für den Widerstand und die Pulsatilität der spiralförmigen Arterien der Gebärmutter.

12. Verfahren nach irgendeinem der Ansprüche 1 bis 11, wobei die Konzentration des in der Endometriumflüssigkeit gelösten Gases im Fundus uteri und/oder im Gebärmutterhalskanal der weiblichen Person gemessen wird.

13. Verfahren nach irgendeinem der Ansprüche 1 bis 12, wobei der Schritt des Messens der Konzentration des in der Endometriumflüssigkeit gelösten Gases durch einen Sensor durchgeführt wird, der in der Lage ist, die Konzentration des in der Endometriumflüssigkeit gelösten Gases zu messen, wobei der Sensor mit dem Endometrium der weiblichen Person nicht in Kontakt kommt.

14. Verfahren nach irgendeinem der Ansprüche 1 bis 13, wobei der Schritt des Messens der Konzentration von gelöstem

# EP 4 429 543 B1

Gas in der Endometriumflüssigkeit in vitro in einer Probe der Endometriumflüssigkeit durchgeführt wird.

15. Verfahren zur Auswahl einer weiblichen Person, die sich *einer assistierten Reproduktionstechnik (ART)* unterzieht, als Kandidatin, um einen Embryo aufzunehmen, wobei das Verfahren die folgenden Schritte umfasst:

   i) Bestimmen, ob das Endometrium der genannten weiblichen Person empfänglich ist, folgend den Verfahrensschritten gemäß irgendeinem der Ansprüche 1 bis 14, und wenn das Endometrium empfänglich ist,
   ii) Auswählen der weiblichen Person als Kandidatin, um einen Embryos in einem Embryotransferverfahren zu empfangen, das so bald wie möglich am selben Tag wie Schritt i) durchgeführt wird.

## Revendications

1. Procédé de détermination de la réceptivité endométriale chez un sujet féminin, le procédé comprenant les étapes suivantes :

   i) mesurer la concentration de gaz dissous dans le liquide endométrial dudit sujet féminin, en obtenant ainsi une valeur de concentration de gaz dissous, le gaz dissous étant choisi parmi le groupe consistant en $O_2$ et $CO_2$,
   ii) comparer ladite valeur de concentration de gaz dissous avec une valeur de référence, une augmentation de la valeur de concentration de l'$O_2$ dissous et/ou une diminution de la valeur de concentration du $CO_2$ dissous par rapport à la valeur de référence est indicative du fait que l'endomètre dudit sujet féminin est réceptif.

2. Procédé selon la revendication 1, dans lequel l'étape de mesure de la concentration de gaz dissous dans le liquide endométrial n'implique pas de contact avec l'endomètre dudit sujet féminin.

3. Procédé selon l'une quelconque des revendications 1 ou 2, dans lequel une variation de la valeur de concentration de gaz dissous par rapport à la valeur de référence est indicative d'un endomètre synchrone pour l'implantation par rapport à un embryon implantable.

4. Procédé selon l'une quelconque des revendications 1 à 3, dans lequel la valeur de référence est la valeur de concentration de l'$O_2$ ou du $CO_2$ dissous dans le liquide endométrial chez ledit sujet féminin, mesurée au moins une fois pendant la période de temps comprise entre 2 jours avant le début de l'ovulation et le jour 2 ou 3 de la phase lutéale.

5. Procédé selon l'une quelconque des revendications 1 à 4, dans lequel la valeur de référence est la valeur de concentration de l'$O_2$ ou du $CO_2$, dissous dans le liquide endométrial chez un autre sujet féminin ou un groupe de sujets féminins, mesurée au moins une fois pendant la période de temps comprise entre 2 jours avant le début de l'ovulation et le jour 2 ou 3 de la phase lutéale.

6. Procédé selon l'une quelconque des revendications 1 à 5, dans lequel la concentration de gaz dissous dans le liquide endométrial est mesurée au moins une fois pendant la période de temps comprise entre 2 jours avant l'ovulation et le jour 12 de la phase lutéale, de manière préférée au moins une fois pendant la période de temps comprise entre le jour de l'ovulation et le jour 10 de la phase lutéale, de manière plus préférée au moins une fois pendant la période de temps comprise entre le jour 2 et le jour 8 de la phase lutéale, de manière encore plus préférée au moins une fois pendant la période de temps comprise entre le jour 3 et le jour 6 de la phase lutéale.

7. Procédé selon l'une quelconque des revendications 1 à 6, dans lequel la concentration de gaz dissous dans le liquide endométrial est mesurée au moins une fois 2 jours avant le début de l'ovulation, au moins une fois au moment de l'ovulation et/ou au moins une fois au jour 2, 4, 6, 8, 10 ou 12 de la phase lutéale.

8. Procédé selon l'une quelconque des revendications 1 à 7, dans lequel la valeur de référence est la valeur de l'$O_2$ dissous à une concentration d'hypoxie physiologique dans le liquide endométrial et l'augmentation de la valeur de concentration de l'$O_2$ dissous par rapport à l'hypoxie physiologique est indicative du fait que l'endomètre dudit sujet féminin est réceptif.

9. Procédé selon l'une quelconque des revendications 1 à 8, dans lequel ledit sujet féminin a entamé un protocole de procréation assistée avec une étape de transfert d'embryon.

**10.** Procédé selon l'une quelconque des revendications 1 à 9, le procédé comprenant en outre, avant l'étape i), une étape de détection de l'ovulation chez ledit sujet féminin en mesurant le taux d'hormone lutéinisante (LH) et/ou par une surveillance par ultrasons.

**11.** Procédé selon l'une quelconque des revendications 1 à 10, le procédé comprenant en outre de mesurer un paramètre choisi parmi le groupe constitué de : la concentration de LH dans l'urine, la concentration de progestérone, d'œstrogène et/ou de VEGF (facteur de croissance endothéliale vasculaire) dans le sang, la présence de follicules et/ou de corps jaune dans les ovaires, l'épaisseur et l'aspect de l'endomètre, et les indices de résistance et de pulsatilité des artères spiralées utérines.

**12.** Procédé selon l'une quelconque des revendications 1 à 11, dans lequel la concentration de gaz dissous dans le liquide endométrial est mesurée dans le fundus de l'utérus et/ou dans le canal cervical dudit sujet féminin.

**13.** Procédé selon l'une quelconque des revendications 1 à 12, dans lequel l'étape de mesure de la concentration de gaz dissous dans le liquide endométrial est réalisée par un capteur capable de mesurer la concentration de gaz dissous dans le liquide endométrial, lequel capteur ne touche pas l'endomètre dudit sujet féminin.

**14.** Procédé selon l'une quelconque des revendications 1 à 13, dans lequel l'étape de mesure de la concentration de gaz dissous dans le liquide endométrial est réalisée in vitro dans un échantillon de liquide endométrial.

**15.** Procédé de sélection d'un sujet féminin soumis à des techniques de procréation assistée (ART) en tant que candidat susceptible de recevoir un embryon, le procédé comprenant les étapes suivantes :

i) déterminer si l'endomètre dudit sujet féminin est réceptif en suivant les étapes du procédé selon l'une quelconque des revendications 1 à 14, et si ledit endomètre est réceptif,
ii) sélectionner ledit sujet féminin en tant que candidat pour recevoir un embryon dans un protocole de transfert d'embryon à mettre en œuvre dès le même jour que l'étape i).

FIG. 1

# Oxygen difussion from blood vessels to the uterine lumen increasses in Secretoy phase

FIG. 2

Assay protocol for the clinical validation of intrauterine pO2 as WOI biomarker

FIG. 3

EP 4 429 543 B1

FIG. 4

FIG. 4 (CONT.)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021028562 A1

**Non-patent literature cited in the description**

- The origin of pre-eclampsia: From decidual "hyperoxia" to late hypoxia. **TRANQUILLI A L et al.** MEDICAL HYPOTHESES. EDEN PRESS, July 2010, vol. 75, 38-46
- Effect of reduced oxygen concentrations on the outcome of in vitro fertilization. **KEA et al.** FERTILITY AND STERILITY. ELSEVIER, 01 December 2006, vol. 87, 213-216
- Oxygen concentration and preimplantation development. **BAVISTER et al.** REPRODUCTIVE BIOMEDICINE ONLINE. ELSEVIER, January 2004, vol. 9, 484-486
- The role of oxygen in ruminant preimplantation embryo development and metabolism. **HARVEY et al.** ANIMAL REPRODUCTION SCIENCE. ELSEVIER SCIENCE PUBLISHERS, February 2007, vol. 98, 113-128
- **BARTELMEZ GW**. The form and the functions of the uterine blood vessels in the rhesus monkey. *Contrib Embryol*, 1957, vol. 36, 154-83
- **NAVOT D. et al.** *Fertil Steril*, 1991, vol. 55, 114-118
- **HARPER MJK**. *Baillieres Clin Obstet Gynaecol*, 1992, vol. 6, 351-371
- **ENCISO, M. et al.** *Sci Rep*, 2021, vol. 11, 13420
- **DOWDY ; WEARDEN**. Statistics for Research. John Wiley and Sons, 1983
- **OTTOSEN et al.** Observations on intrauterine oxygen tension measured by fiber-optic microsensors. *Reproductive biomedicine online*, 2006, vol. 13 (3), 380-5